# EUROPEAN PATENT SPECIFICATION

(11) **EP 0 541 651 B1**
(45) Date of publication and mention of the grant of the patent: **20.04.1994**
(21) Application number: 91914112.7
(22) Date of filing: 01.08.1991
(51) Int. Cl.: A61B 17/60

(54) **ORTHOPAEDIC MANIPULATOR FOR REDUCTION AND EXTERNAL FIXATION**
ORTHOPÄDISCHER MANIPULATOR ZUM KNOCHENRICHTEN UND ÄUSSERE FIXIERUNG
MANIPULATEUR ORTHOPEDIQUE POUR LA REDUCTION ET LA FIXATION EXTERNE

(30) Priority: 03.08.1990 IT 4043490
(43) Date of publication of application: 19.05.1993
(73) Proprietor: JAQUET ORTHOPEDIE S.A., CH-1228 Plan-les-Ouates (CH)
(72) Inventor: Campopiano, Ascanio, I-80059 Torre del Greco (IT)
(74) Representative: Dietlin, Henri
(86) International application number: PCT/IT91/00071
(87) International publication number: WO 92/02184

(56) References cited:
- EP-A- 0 177 270
- EP-A- 0 248 138
- DE-A- 3 305 597

## Description

The method of external bone fixation for the therapy of skeletal fractures of limbs has been in use for many years in traumatologic medicine.

Such a method often substitutes the use of immobilizing plastering apparatuses which always involve a long rehabilitation stage and, at the same time, often avoids an operation in open air.

However, it is often the difficulty in practising reduction manoeuvers that allow an optimum anatomic reconstruction of the fractured skeletal segment that limits its utilization.

The various commercially available external fixer systems do not allow such manoeuvers, or they allow them only partially, often with the risk of making the already obtained result worse, and often with a complicated an approximative mechanics.

From the document EP-A-0.177.270 there is known a fracture reduction system which is functionally closest to the subject-matter of claim 1. However, the structure of the known device is entirely different. While in the device of claim 1 all components are arranged with respect to the adjustable circular sector comprising two half-sectors, in the known device rotation units are mounted on a telescopic bar.

As far as the structural elements are concerned, document EP-A-0.248.138 represents the closest prior art. In the known bone fixation device a sliding block is provided in a housing having the shape of a circular sector. External bone fixation devices comprising clamps for holding bone screws are connected by means of respective ball joints to a respective rod, which is fixed to either the housing or the sliding block.

The object of the present patent for industrial invention is to suggest a means that makes the reduction manoeuvers easier allowing micrometric displacement of the bone fragments to be reduced.

However it allows the manoeuvers to be practised independently of each other, so as not to compromise the already obtained reduction result.

The present invention concerns a manipulator for two external fixation devices comprising clamps for holding bone screws, said manipulator being made up of a circular sector that is adjustable to comprise an angular range of from 60° to 120°, and is assembled of two half-sectors arranged for sliding movement on each other to adjust the angular range of the circular sector, one extremity of each of the two half-sectors being provided with a seat containing a cylindrical bush with a portion of the cylindrical surface of the bush longitudinally toothed to mate with an endless screw for rotation of the bush, said bush being also in engagement with a screw for axial displacement of the bush in its seat, each bush having a stem projecting therefrom for connection to the respective clamps of one of said fixation devices, said two half-sectors being arranged such that there is a bush at the opposite ends of the circular sector.

In the preferred embodiment, the axes of the bushes lie in a same plane and, in use, converge to the focus of the fracture, allowing the rotations of bone fragments about axes converging into the fracture focus.

The manipulator is realized to be removed or to remain applied to a limb through the proper fixers, and to his end it is endowed with clamping members that ensure a stable maintenance of the reached position.

According to the suggested solution idea the realized manipulator allows, by acting upon proper screw handlebar grips, a micrometric displacement of the fractured bone fragments in all the possible directions realizing all the degrees of freedom and the relative axial and angular rotations.

The manipulator is shown in an embodiment thereof given as a matter of example and not of limitation in the annexed plates of drawings.

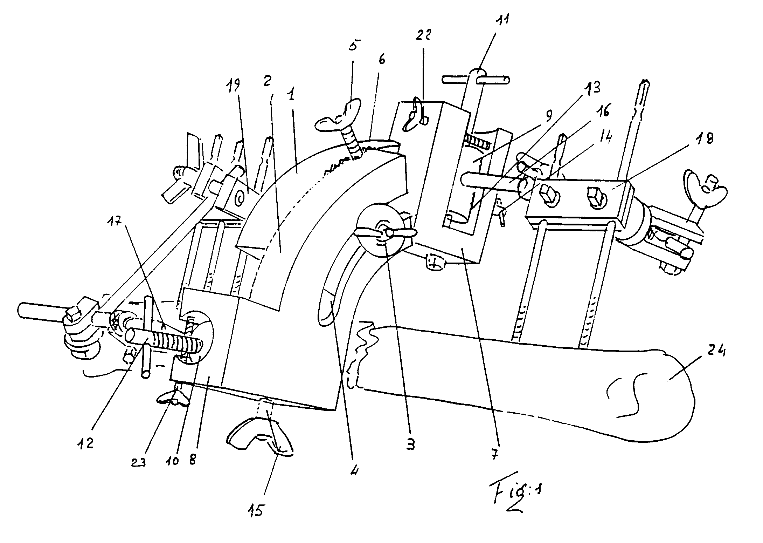

Plate I presents it in Figure 1 in an axonometric front side view that shows it in the stage of its utilization applied to a fixer of a known type.

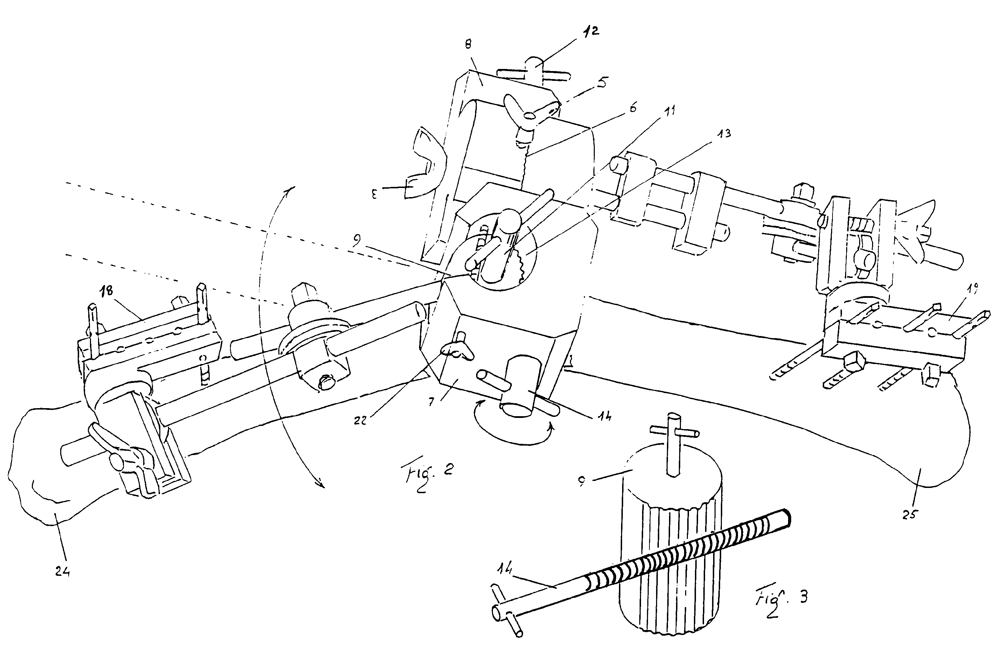

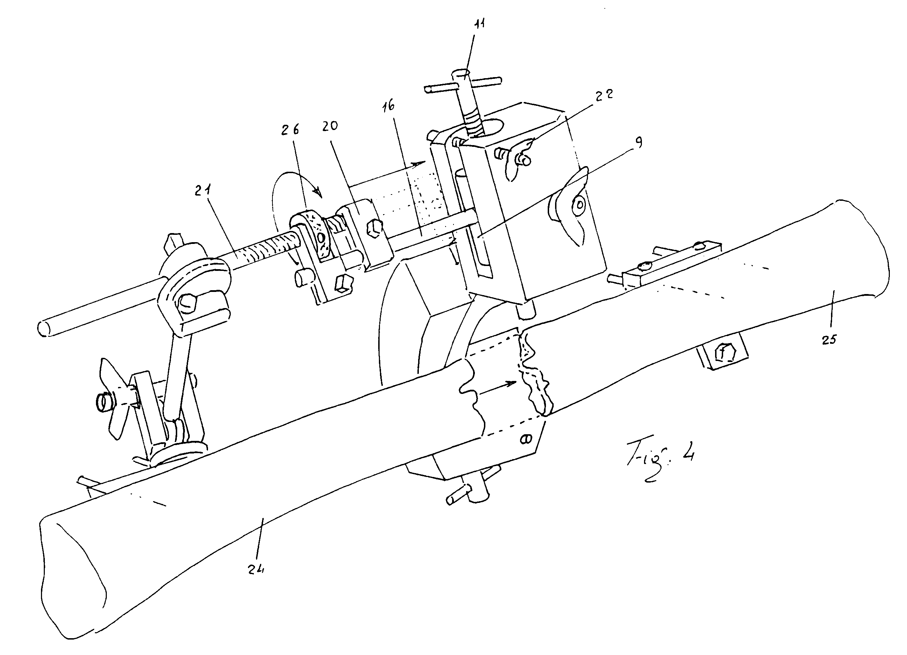

Plate II in Figure 2 puts into evidence in a top axonometric view the maneuver of angular rotation in the horizontal plane made possible by the manipulator; in Figure 3 it illustrates the particular mating between the toothed sector and the endless screw that allows some rotation manoeuvers.

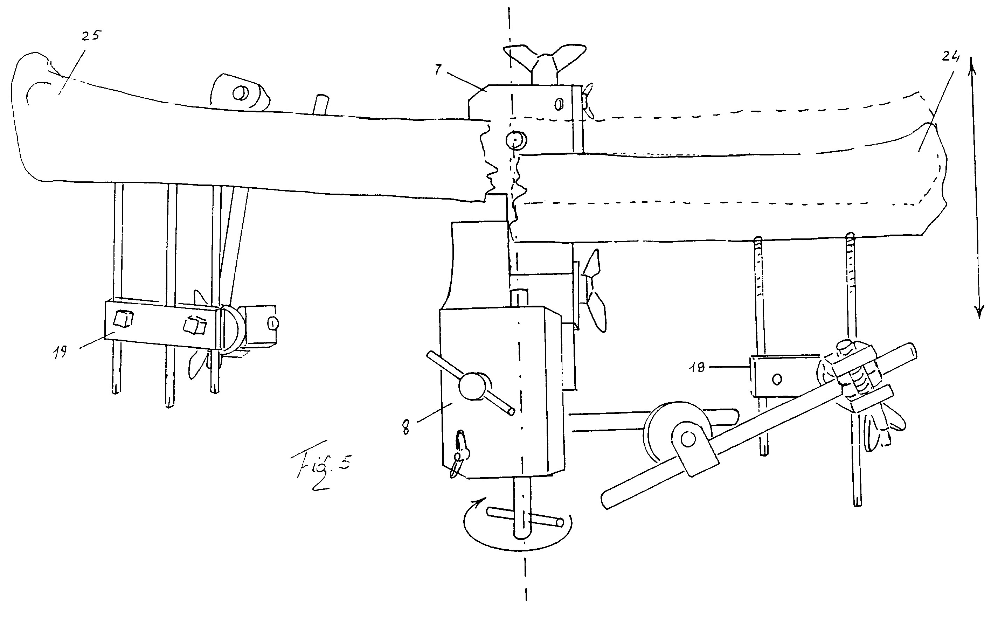

Plate III in Figure 4 shows in a rear axonometric view the possible maneuver for the axial slide of the fragments.

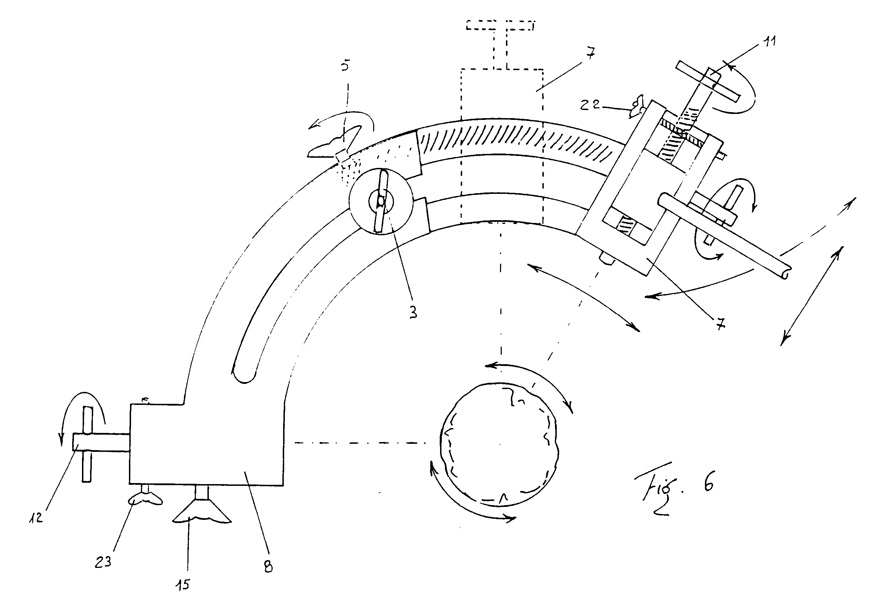

Plate IV in Figure 5 represents the manipulator in a rear axonometric view that puts into evidence the manoeuver for the transverse slide in the horizontal plane.

Plate V, finally represents in Figure 6 a front view of the detail of the sector that allows the axial rotation of the fragments.

With reference to the figures, the manipulator is made up of a sector that extends by a quarter of a circle and is comprised of two portions 1 and 2 endowed with guides fit for sliding angularly on each other when the screw 3 that is located in the slit 4 which goes through a large part of the sector 2 is loosened.

In this way the sector can be reduced, assuming angles that range from about 60° to about 120°.

The screw 5 that engages with the toothing 6 provided on a portion of the contact faces of the two sectors allows a micrometric displacement of a sector with respect to the other.

Each sector at the not engaged extremity projects respectively into the blocks 7 and 8, so shaped as to receive the bushes 9 and 10 displaced by the screws 11 and 12 and endowed along a portion of their cylindrical surface with toothings 13 that engage respectively with endless screws 14 and 15 slightly angled with respect to the orthogonal axis of the screw itself, as put into evidence in Figure 3.

From bushes 9 and 10 stems 16 and 17 project, which by means of proper transmissions end at the clamps of the fixers 18 and 19.
On the stem 17 the slab 20 is provided of the fixer which connects it to the slide bar of the fixer 21 or other axial slide system.

Such a manoeuver, realized through a slide bar of the known fixer, will be able to be realized with any other slide system that acts along the longitudinal axis of the bar of the manipulator.

The blocks 7 and 8 are moderately elastic and endowed with transverse screws 22 and 23 fit to tighten them in order to clamp the motion of the internal bushes.

From what has been illustrated it is apparent that by acting upon the described screws the following displacements of the bone fragments 24 and 25 connected to the relevant clamps of the fixer can be performed:
a) by acting upon the screw 26 of the fixer or other known axial slide system one determines the approaching or the removal of the fragments along the longitudinal axis;
b) by acting upon the screw 12 one determines the transverse translation in the vertical plane of the fragment 24 with respect to the fragment 25;
c) by acting upon the screw 11 one determines the transverse translation in the horizontal plane of the fragment 25 with respect to the fragment 24;
d) by acting upon the screws 14 and 15 one causes the relative angle of the fragments in the vertical and horizontal plane;
e) By acting upon the screw 5, finally, one determines the angular displacement of the two half - sectors 1 and 2 and thus the rotation of the fragments about their own axis.

The geometric characteristics that are to be respected in the realization of the fixer are: the coplanarity of the axes of the screws 11 and 12 and their convergence into the fracture focus.

When the fracture has been reduced, the screws 3 and the screws 22 and 23 are tightened, so as to clamp the manipulator in the last reached position; in this position it can remain applied to the limb for possible subsequent adjusting manoeuvers. This is made possible by its reduced size and by its extremely restricted weight.

It is however possible to remove it also by clamping the fixers with a simple connector of a known type.

The light weight and smallest encumbrance characteristics are a consequence of the solutions adopted for the realization of all the movements that occur according to the suggested solution idea with an extremely compact instrument.

Formal and structural variations can be made to the solution idea, without departing from the scope of the invention, which is defined by the following claims.

## Claims

1. A manipulator for two external fixation devices (18, 19) comprising clamps for holding bone screws, said manipulator being made up of a circular sector that is adjustable to comprise an angular range of from 60° to 120°, and is assembled of two half-sectors (1,2) arranged for sliding movement on each other to adjust the angular range of the circular sector, one extremity of each of the two half-sectors being provided with a seat (7, 8) containing a cylindrical bush (9, 10) with a portion of the cylindrical surface of the bush longitudinally toothed to mate with an endless screw (14, 15) for rotation of the bush, said bush being also in engagement with a screw (11, 12) for axial displacement of the bush in its seat, each bush having a stem (16, 17) projecting therefrom for connection to the respective clamps of one of said fixation devices, said two half-sectors being arranged such that there is a bush at the opposite ends of the circular sector.

2. A manipulator according to the preceding claim, characterized in that the axes of the bushes lie in a same plane and, in use, converge to the focus of the fracture.

3. A manipulator according to the preceding claims, characterized in that the axial motion of the bushes through proper screws ensures the translation in the horizontal and vertical plane of the bone fragments connected to the manipulator through clamps of the fixer connected to the bushes themselves.

4. A manipulator according to the preceding claims, characterized in that by acting upon the endless screws with the axis slightly angled with respect to that of the bushes that mate therewith the angulation of the fragments in the horizontal and in the vertical plane is ensured.

5. A manipulator according to the preceding claims, characterized in that the slide of the half-sectors by means of proper micrometric screws allows the rotation of the bone fragments about their own axis.

6. A manipulator according to the preceding claims, characterized in that proper screws clamp both the seats and the circular sector once the desired position of the bone fragments has been reached.

## Patentansprüche

1. Manipulator für zwei äußere Fixiervorrichtungen (18, 19), mit Klammern zum Halten von Knochenschrauben, wobei der Manipulator aus einem kreisförmigen Sektor besteht, der so einstellbar ist, daß ein Winkelbereich von 60° bis 120° umfaßt wird, und aus zwei Halbsektoren (1, 2) aufgebaut ist, die für die Gleitbewegung aufeinander angeordnet sind, um den Winkelbereich des kreisförmigen Sektors einzustellen, wobei ein äußeres Ende jedes der beiden Halbsektoren mit einer Lagerung (7, 8) versehen ist, die eine zylindrische Buchse (9, 10) enthält, wobei ein Teil der zylindrischen Fläche der Buchse in Längsrichtung gezahnt ist, um an eine Endlosschraube (14, 15) zum Drehen der Buchse zu passen, wobei die Buchse auch in Eingriff mit einer Schraube (11, 12) für die axiale Verlagerung der Buchse in ihrer Lagerung ist, wobei jede Buchse eine Stange (16, 17) hat, die von ihr für die Verbindung mit der jeweiligen Klammer einer der Fixiervorrichtungen hervorragt, wobei die beiden Halbsektoren so angeordnet sind, daß es eine Buchse an den gegenüberliegenden Enden des kreisförmigen Sektors gibt.

2. Manipulator nach dem vorangehenden Anspruch, dadurch gekennzeichnet, daß die Achsen der Buchsen in einer gemeinsamen Ebene liegen und bei der Verwendung auf das Zentrum des Bruches zusammenlaufen.

3. Manipulator nach den vorangehenden Ansprüchen, dadurch gekennzeichnet, daß die axiale Bewegung der Buchsen durch geeignete Schrauben die Verschiebung in der Horizontal- und Vertikalebene der Knochenfragmente sicherstellt, die mit dem Manipulator über Klammern des Fixierers verbunden sind, die selbst mit den Buchsen verbunden sind.

4. Manipulator nach den vorangehenden Ansprüchen, dadurch gekennzeichnet, daß durch Wirken auf die Endlosschrauben, wobei die Achsen leicht in bezug auf die der Buchsen, die an sie angepaßt sind, gewinkelt sind, die Winkelbewegung der Fragmente in der Horizontal- und in der Vertikalebene sichergestellt wird.

5. Manipulator nach den vorangehenden Ansprüchen, dadurch gekennzeichnet, daß das Gleiten der Halbsektoren mittels geeigneter Mikrometerschrauben die Drehung der Knochenfragmente um ihre eigene Achse erlaubt.

6. Manipulator nach den vorangehenden Ansprüchen, dadurch gekennzeichnet, daß die geeigneten Schrauben sowohl die Lagerungen als auch den kreisförmigen Sektor verklemmen, wenn einmal die gewünschte Position der Knochenfragmente erreicht worden ist.

## Revendications

1. Manipulateur de deux dispositifs (18,19) de fixation externe comportant des pinces de serrage des fiches osseuses, ledit manipulateur comportant un secteur circulaire réglable pour couvrir un champ angulaire de l'ordre de 60° à 120° environ et composé de deux demi-secteurs (1,2) aptes à glisser l'un sur l'autre pour ajuster le champ angulaire du secteur circulaire, une extrémité de chacun des deux demi-secteurs étant munie d'un siège (7,8) contenant une douille cylindrique (9,10), une partie de la surface cylindrique de la douille présentant des dentures longitudinales aptes à coopérer avec une vis sans fin (14,15) d'entraînement de la douille en rotation, cette douille coopérant également avec une vis (11,12) de déplacement axial de la douille dans son siège, chaque douille comportant une tige (16,17) qui s'en projette pour la liaison aux pinces de l'un des deux dispositifs de fixation, les deux demi-secteurs étant disposés pour qu'il y ait une douille aux extrémités opposées du secteur circulaire.

2. Manipulateur selon la revendication précédente, caractérisé en ce que les axes des douilles sont dans un même plan et convergent vers le foyer de la fracture, en cours d'utilisation.

3. Manipulateur selon les revendications précédentes, caractérisé en ce que le mouvement axial des douilles par les vis appropriées assure la translation dans les plans horizontal et vertical des fragments d'os reliés au manipulateur par des pinces du fixateur relié aux douilles elles-mêmes.

4. Manipulateur selon les revendications précédentes, caractérisé en ce qu'en agissant sur les vis sans fin, dont l'axe a une légère inclinaison par rapport à l'axe de la douille correspondante, on peut orienter angulairement les fragments dans le plan horizontal et dans le plan vertical.

5. Manipulateur selon les revendications précédentes, caractérisé en ce que le mouvement relatif de glissement relatif des deux demi-secteurs, par des vis micrométriques appropriées, permet la rotation des fragments osseux sur leur propre axe.

6. Manipulateur selon les revendications précédentes, caractérisé en ce que des vis appropriées permettent de bloquer les sièges et les secteurs circulaires associés une fois que la position désirée des fragments osseux a été atteinte.
